# FASCICULE DE BREVET EUROPEEN

(11) **EP 3 082 831 B1**
(45) Date de publication et mention de la délivrance du brevet: **06.01.2021**
(21) Numéro de dépôt: 14827505.0
(22) Date de dépôt: 12.12.2014
(51) Int. Cl.: A61K 31/724, A61P 9/00, A61P 9/10, A61P 43/00, A61P 3/00, A61P 3/06, A61P 7/00, A61P 25/00, A61P 25/28, A61P 25/16

(54) **COMPOSITIONS A BASE DE METHYL-CYCLODEXTRINES POUR LE TRAITEMENT ET/OU LA PREVENTION DE MALADIES PAR AUGMENTATION DU TAUX DE CHOLESTEROL-HDL**
METHYL-CYCLODEXTRIN ENTHALTENDE ZUSAMMENSETZUNGEN ZUR BEHANDLUNG ODER VORBEUGUNG VON KRANKHEITEN DURCH ERHÖHUNG DES HDL-CHOLESTEROLSPIEGELS
COMPOSITIONS COMPRISING METHYL-CYCLODEXTRINES TO TREAT OR PREVENT DISEASES BY ENHANCEMENT OF THE HDL-CHOLESTEROL LEVEL

(30) Priorité: 13.12.2013 FR 1362633
(43) Date de publication de la demande: 26.10.2016
(60) Demande divisionnaire: 19184268.1
(73) Titulaire: Roquette Frères, 62136 Lestrem (FR)
(72) Inventeur: SALOME, Marc, F-31320 Mervilla (FR); WILS, Daniel, F-59190 Morbecque (FR); PARISSAUX, Xavier, F-62500 Saint Omer (FR); MACH, François, CH-1207 Geneve (CH); MONTECUCCO, Fabrizio, CH-1206 Geneve (CH)
(74) Mandataire: Plasseraud IP
(86) Numéro de dépôt international: PCT/FR2014/053309
(87) Numéro de publication internationale: WO 2015/087016

(56) Documents cités:
- WO-A1-02/43742
- US-A1- 2008 032 925
- SWAROOP ET AL.: "EVALUTATION OF CHOLESTEROL REDUCTION ACTIVITY OF METHYL-BETA-CYCLODEXTRIN USING DIFFERENTIATED HUMAN NEURONS AND ASTROCYTES", JOURNAL OF BIOMOLECULAR SCREENING, vol. 17, no. 9, 2012, pages 1243-1251, XP002726510,
- BUSSEUIL ET AL.: "A STUDY OF THE EFFECT OF METHYL-BETA-CYCLODEXTRIN ON CHOLESTEROL CONTENT AND VASCULAR RESPONSES OF THE PORCINE ISOLATED CORONARY ARTERY", BRITISH JOURNAL OF PHARMACOLOGY, vol. 131, no. PROC. SUP., 2000, page 10P, XP002726511,

### SEQUENCE LISTING

<110> ROQUETTE FRERES
<120> COMPOSITIONS A BASE DE METHYL-CYCLODEXTRINES POUR LE TRAITEMENT ET/OU LA PREVENTION DE MALADIES PAR AUGMENTATION DU TAUX DE CHOLESTEROL-HDL
<130> B1729PC00
<160> 6
<170> PatentIn version 3.5
<210> 1
   <211> 19
   <212> DNA
   <213> artificial sequence
<220>
   <223> primer
<400> 1
   gtgtctcgcc tgttctcag 19
<210> 2
   <211> 19
   <212> DNA
   <213> artificial sequence
<220>
   <223> primer
<400> 2
   gaaacatcac ctcctgccg 19
<210> 3
   <211> 22
   <212> DNA
   <213> artificial sequence
<220>
   <223> primer
<400> 3
   gaggaagaaa ggatacaaga cc 22
<210> 4
   <211> 22
   <212> DNA
   <213> artificial sequence
<220>
   <223> primer
<400> 4
   gtcagtatct ccttgaccat tt 22
<210> 5
   <211> 22
   <212> **DNA**
   <213> artificial sequence
<220>
   <223> primer
<400> 5
   cccttaatcc acctcatcaa tc 22
<210> 6
   <211> 22
   <212> DNA
   <213> artificial sequence
<220>
   <223> primer
<400> 6
   gaagtttttg acccctgtga ac 22

## Description

La présente invention est relative à des compositions pharmaceutiques permettant d'augmenter le taux de cholestérol-HDL chez un individu. La présente invention concerne plus spécifiquement l'utilisation d'une composition pharmaceutique dans le traitement et/ou la prévention de maladies susceptibles d'être traitées et/ou prévenues par une augmentation du taux de cholestérol-HDL, ou dans le traitement et/ou la prévention de l'athérosclérose ou des complications liées à un athérome, ou des maladies du système nerveux central.

### ARRIERE-PLAN TECHNOLOGIQUE DE L'INVENTION

Le cholestérol est un lipide de la famille des stérols jouant un rôle central dans de nombreux processus biochimiques.

C'est un composant majeur des membranes cellulaires animales qui contribue à leur stabilité et au maintien de leur structure en s'intercalant entre les phospholipides. En effet, le cholestérol rigidifie la membrane en empêchant sa gélification car il évite la cristallisation des acides gras et diminue également la perméabilité membranaire aux molécules hydrosolubles. En s'intercalant dans les membranes, le cholestérol permet également la formation de radeaux lipidiques qui sont des zones essentielles à l'ancrage de protéines fonctionnelles.

Par ailleurs, le cholestérol est aussi retrouvé dans les neurones où il permet la synthèse de neurotransmetteurs par exocytose et donc la propagation du message nerveux. Il est directement impliqué dans la formation de peptides beta-amyloïde, et donc dans la pathogenèse de la maladie d'Alzheimer. Une association entre l'athérosclérose et la maladie d'Alzheimer a aussi été démontrée (Mikael Simons and al., « Cholesterol depletion inhibits the generation of b-amyloid in hippocampal neurons », Proc. Natl. Acad. Sci. USA ; Vol. 95, pp. 6460-6464, May 1998, Neurobiology.)

Le métabolisme du cholestérol est également précurseur de nombreuses molécules comme par exemple les hormones stéroïdes (cortisol, cortisone et aldostérone), les hormones stéroïdes sexuelles (progestérone, oestrogènes et testostérone), la vitamine D3, l'hème A, les protéines prénylées ou farnésylées, l'ubiquinone ou coenzyme Q10, le dolichol, le facteur nucléaire NF kappa B, la protéine Tau et les sels biliaires.

Le cholestérol est véhiculé dans le sang par des systèmes de transport aux rôles très différents, à savoir les lipoprotéines LDL (lipoprotéines de petite densité) et les lipoprotéines HDL (lipoprotéines de haute densité).

Le HDL-cholestérol est considéré comme protecteur vis-à-vis des maladies cardiovasculaires et est fréquemment désigné comme « bon cholestérol ».

Les LDL transportant le cholestérol pénètrent dans la paroi artérielle puis sont captées par les monocytes et macrophages résidant dans cette paroi artérielle ainsi que par les cellules musculaires lisses. Il s'ensuit une charge excessive en lipides des macrophages et cellules musculaires lisses qui se transforment alors en cellules spumeuses à l'origine des processus d'athérosclérose et correspondant au premier stade de la formation de la plaque d'athérome. Il est alors question de « mauvais cholestérol ».

L'athérome est défini par l'Organisation Mondiale de la Santé comme une "association de remaniement de l'intima des artères de gros et moyen calibre consistant en une accumulation focale de lipides, glucides complexes, de sang et de dépôts calcaire, avec remaniements de la média". L'athérome est à l'origine de la majorité des maladies cardiovasculaires et est la principale cause de morbidité et de mortalité des pays industrialisés. L'athérome débute par la formation d'une « strie lipidique », simple dépôt de graisse, linéaire et sans conséquence pour le flux, située entre endothélium et média de l'artère. Avec le temps, cette strie peut grossir, se charger en lipides, en fibrogène, en plaquettes et autres cellules sanguines et en calcium pour constituer la « plaque d'athérome ». Celle-ci devient plus ou moins importante et peut diminuer suffisamment le calibre de l'artère pour diminuer son débit.

Il existe deux types de complications : la première, d'évolution longue, est due à sa croissance lente, gênant de plus en plus le passage du sang jusqu'à l'empêcher totalement par obstruction. La seconde, rapide et responsable des complications aigües, consiste en la lésion ou la rupture de l'endothélium : la brèche formée est alors obstruée par une agrégation des plaquettes sanguines et la formation d'un caillot sanguin qui peut rapidement totalement obstruer le vaisseau. Ce caillot peut également se détacher et obstruer plus en aval. La plaque peut également se détacher partiellement et obstruer également l'artère, ou plus rarement, libérer son contenu et faire une embolie de cholestérol. Enfin, la dilatation de la paroi artérielle induite par l'augmentation de volume de la plaque peut aboutir à la formation d'un anévrisme, avec risque de rupture.

La classe pharmaceutique la plus employée pour prévenir l'athérome sont les statines, qui visent à réduire le taux de cholestérol-LDL et de lipides circulants, en complément d'un régime adapté. Cependant l'intérêt et l'utilité des statines sont depuis quelques temps vivement critiqués par certains chercheurs et cliniciens qui affirment que les statines, après analyse d'essais statistiques controversés car contradictoires, n'apportent pas aux patients le bénéfice thérapeutique escompté. A cette mise en cause s'ajoutent les effets secondaires néfastes, et même parfois dramatiques, qui ont été identifiés et ont conduit dans certains cas à un retrait du marché.

Les autres traitements médicamenteux proposés aujourd'hui sont :
- les antiagrégants plaquettaires comme l'aspirine ou le clopidogrel, censés diminuer la constitution de caillots à partir de la plaque d'athérome ;
- les médicaments antihypertenseurs tels que les inhibiteurs de l'enzyme de conversion de l'angiotensine.

Les traitements pharmaceutiques qui existent à l'heure actuelle visent donc uniquement à diminuer les risques liés à l'athérome. Aucun des médicaments existant sur le marché ne s'attaque directement à la plaque d'athérome.

Lorsque le pronostic vital est en jeu, le traitement est chirurgical ou endovasculaire. Il vise à restaurer la lumière artérielle, à irriguer le territoire privé d'oxygène ou à supprimer l'anévrisme artériel. Parmi ces techniques, on peut citer l'angioplastie, la désobstruction par endartériectomie, le pontage.

Finalement, le principal moyen de lutte contre l'athérome et ses complications reste encore aujourd'hui d'ordre comportemental : arrêt du tabac, développement de l'activité physique, contrôle de la tension artérielle, correction d'une dyslipidémie, équilibre du diabète, régime.

Ainsi, force est de constater qu'il existe clairement et depuis longtemps, un besoin non satisfait de traitement et/ou de prévention de l'athérome.

Diverses approches ont pourtant été tentées, sans qu'aucune solution nouvelle n'ait pu être retenue. Le cholestérol-HDL en particulier a commencé à être étudié en 1975 lorsque des chercheurs ont mis en évidence la relation entre des taux de cholestérol-HDL élevés et la diminution de l'incidence des maladies cardio-vasculaires (Rye K.A. 2013. High density lipoprotein structure, function, and metabolism: a new Thematic Series. J. Lipid Res. 54:(8) 2031-2033). Les résultats positifs de ces études ont encouragé le développement de traitements visant à augmenter les taux de cholestérol-HDL, parmi lesquelles on retiendra les inhibiteurs de l'enzyme CETP, (protéine de transfert des esters de cholestérol) qui ont été les tous premiers agents développés et évalués à grande échelle, spécifiquement dans ce but. Cependant aucun de ces traitements n'a permis de réduire la survenue des accidents cardiovasculaires.

Busseuil et al. (Brit. J. Pharmacol., 2000, 131, 10P) ont rapporté une étude de l'effet de la méthyl-β-cyclodextrine (MβCD) sur la teneur en cholestérol et les réponses vasculaires de l'artère coronaire isolée porcine, qui montre qu'un prétraitement pendant la nuit avec de la MβCD peut considérablement réduire la teneur en cholestérol des artères coronaires.

Swaroop et al. (J. Biomol. Screen., 2012, 17, 1243-1251) ont décrit une évaluation de l'activité de réduction de cholestérol de la MβCD qui montre que la MβCD réduit l'accumulation de cholestérol lysosomal dans les fibroblastes cutanés dérivés de patients atteints de la maladie de Niemann Pick de type C.

Finalement, il n'existe toujours pas sur le marché de médicament capable d'induire efficacement une augmentation du taux de cholestérol-HDL chez le patient, notamment parce que les mécanismes impliqués sont aujourd'hui encore loin d'être élucidés. En particulier, il n'existe pas sur le marché de médicament capable de lutter efficacement et activement contre des maladies susceptibles d'être traitées et/ou prévenues par une augmentation du taux de cholestérol-HDL. En outre, il existe un besoin constant de médicament capable de lutter contre l'athérosclérose, en particulier contre la formation de la plaque d'athérome.

### RESUME DE L'INVENTION

Il est du mérite de la Demanderesse d'avoir découvert que des compositions pharmaceutiques à base de méthyl-cyclodextrine présentant un degré de substitution molaire particulier compris entre 0,05 et 1,5 permettent d'induire une augmentation du taux de cholestérol-HDL, en particulier du taux de cholestérol-HDL plasmatique, et sont donc extrêmement intéressantes pour le traitement ou la prévention de maladies susceptibles d'être traitées et/ou prévenues par une augmentation du taux de cholestérol-HDL, notamment de l'athérosclérose ou des complications liées à un athérome, ou des maladies du système nerveux central.
L'objet de la présente invention est donc de proposer une composition pharmaceutique comprenant au moins une méthyl-cyclodextrine présentant un degré de substitution molaire compris entre 0,4 et 0,9 pour son utilisation dans le traitement et/ou la prévention de maladies susceptibles d'être traitées et/ou prévenues par une augmentation du taux de cholestérol-HDL.
Un autre objet de la présente invention est de proposer une composition pharmaceutique comprenant au moins une méthyl-cyclodextrine présentant un degré de substitution molaire compris entre 0,4 et 0,9 pour son utilisation dans le traitement et/ou la prévention de l'athérosclérose ou des complications liées à un athérome, ou des maladies du système nerveux central.
Préférentiellement, les compositions de l'invention sont utilisées dans le traitement et/ou la prévention de l'athérosclérose ou des complications liées à un athérome. Les complications liées à un athérome comprennent typiquement l'ischémie, par exemple l'ischémie du myocarde, les maladies coronariennes, l'angine de poitrine, le syndrome coronarien aigu, l'infarctus du myocarde, l'infarctus mésentérique, l'accident vasculaire-cérébral, l'anévrisme, ou l'artériopathie des membres inférieurs et leurs conséquences (conséquences liées à hypopoxie/ischémie, par exemple un diabète consécutif à athérosclérose).
Dans un autre mode préféré, les compositions de l'invention utilisées dans le traitement et/ou la prévention des maladies du système nerveux central sont choisies parmi la maladie d'Alzheimer, la maladie de Parkinson et les maladies lysosomales affectant le système nerveux central. Préférentiellement, les maladies lysosomale affectant le système nerveux central sont choisies parmi la maladie de Niemann-Pick, préférentiellement la maladie de Niemann-Pick type A, la maladie de Niemann-Pick type B, ou la maladie de Niemann-Pick type C.
La méthyl-cyclodextrine utilisée dans les compositions pharmaceutiques de l'invention présente un degré de substitution molaire compris entre 0,4 et 0,9. Avantageusement, le degré de substitution molaire est compris entre 0,6 et 0,8. Dans un mode de réalisation particulier, la méthyl-cyclodextrine est une méthyl-β-cyclodextrine. Dans un autre mode de réalisation particulier, la méthyl-cyclodextrine est substituée sur l'hydroxyle porté par le carbone C2 des unités glucopyranoses, ou par les carbones C3 et/ou C6 des unités glucopyranoses ou par une combinaison des carbones C2, C3 et/ou C6, de préférence C2 et C6 des unités glucopyranoses.
De préférence, les compositions de méthyl-cyclodextrines comprennent une ou plusieurs méthyl-β-cyclodextrines choisies parmi le groupe consistant en des méthyl-β-cyclodextrines substituées sur l'hydroxyle porté par le carbone C2 des unités glucopyranoses, des méthyl-β-cyclodextrines substituées sur l'hydroxyle porté par les carbones C3 et/ou C6 des unités glucopyranoses, des méthyl-β-cyclodextrines substituées sur l'hydroxyle porté par les carbones C2, C3 et/ou C6, de préférence C2 et C6 des unités glucopyranoses et lesdites méthyl-β-cyclodextrines présentant un degré de substitution molaire compris entre 0,6 et 0,8.

Avantageusement, les compositions de méthyl-cyclodextrines comprennent au moins 50, 60, ou 75 % de méthyles substituées sur l'hydroxyle porté par le carbone C2 des unités glucopyranoses.

Facultativement, les compositions comprenant au moins une méthyl-cyclodextrine comprennent également une cyclodextrine, en particulier β -cyclodextrine, non substituée et/ou une cyclodextrine, en particulier β -cyclodextrine, substituée par des groupes sulfobutyléther (SBE-) et hydroxypropyles (HP-), de préférence avec un degré de substitution molaire compris entre 0,05 et 1,5.

Les compositions pharmaceutiques utilisées dans le cadre de la présente invention peuvent comprendre en outre au moins un agent actif supplémentaire.
L'agent actif supplémentaire est préférentiellement un agent actif utilisé dans le traitement de l'athérosclérose ou des complications liés à un athérome, préférentiellement,
- une statine,
- un agent antiagrégant plaquettaire ou anticoagulant, de préférence sélectionné parmi l'aspirine, le clopidogrel, les nouveaux anticoagulants oraux comme le dabigatran, l'apixaban, le rivaroxaban, et les anti-vitamines K
- un agent antihypertenseur, de préférence sélectionné parmi les inhibiteurs de l'enzyme de conversion de l'angiotensine comme le perindopril, le captopril, l'enalapril, le lisinopril ou le ramipril, les antagonistes du récepteur à l'angiotensine II comme le losartan, le valsartan, ou le candesartan, et/ou parmi les bêta-bloquants comme l'acébutolol, le labétalol, le nadolol, l'oxprénolol, le penbutolol, le pindolol, ou le propranolol,
- ou une combinaison de ceux-ci.

De manière préférée, l'agent actif supplémentaire est un antagoniste du récepteur à l'angiotensine II, comme le losartan, le valsartan, ou le candesartan.
Les compositions pharmaceutiques selon l'invention sont susceptibles d'être administrées par voie orale, par voie parentérale, ou par voie cutanée ou mucosale.

Dans un mode de réalisation particulier, les compositions de l'invention sont en outre utilisées pour favoriser une diminution du taux de triglycérides et d'acides gras circulants.

Dans un autre mode de réalisation particulier, les compositions de l'invention sont utilisées pour réduire ou prévenir les plaques d'athérome.

Encore dans un autre mode de réalisation particulier, les compositions utilisées dans l'invention sont exemptes ou substantiellement exemptes de vésicules de phospholipides.

### DESCRIPTION DETAILLEE DE L'INVENTION

Les inventeurs ont identifié une nouvelle utilisation d'une composition pharmaceutique comprenant au moins une méthyl-cyclodextrine présentant un degré de substitution molaire compris entre 0,4 et 0,9 dans le traitement et/ou la prévention de maladies susceptibles d'être traitées et/ou prévenues par une augmentation du taux de cholestérol-HDL et/ou par une réduction ou prévention des plaques d'athérome.
En particulier, les inventeurs ont découvert de façon surprenante que la composition de l'invention avait pour effet d'augmenter le taux de cholestérol-HDL chez un individu, et en outre, de diminuer le taux de triglycérides et d'acides gras circulants et de réduire les plaques d'athérome. Les inventeurs ont ainsi démontré que la composition de l'invention grâce à ses différentes propriétés peut notamment être utilisée dans le traitement et/ou la prévention de maladies liées à une surcharge, et/ou à un stockage, et/ou à l'accumulation de cholestérol dans les tissus, ainsi que leurs conséquences. En particulier, ces maladies comprennent l'athérosclérose ou des complications liées à un athérome, et les maladies du système nerveux central, notamment la maladie d'Alzheimer, de Parkinson ou de Niemann Pick type C.

L'invention concerne donc une composition pharmaceutique comprenant au moins une méthyl-cyclodextrine présentant un degré de substitution molaire compris entre 0,4 et 0,9 pour son utilisation dans le traitement et/ou la prévention de maladies susceptibles d'être traitées et/ou prévenues par une augmentation du taux de cholestérol-HDL, en particulier l'athérosclérose ou des complications liées à un athérome, et/ou les maladies du système nerveux central, notamment la maladie d'Alzheimer, de Parkinson ou de Niemann Pick type C. Elle concerne également une composition pharmaceutique comprenant au moins une méthyl-cyclodextrine présentant un degré de substitution molaire compris entre 0,05 et 1,5 pour son utilisation dans le traitement et/ou la prévention de maladies susceptibles d'être traitées et/ou prévenues par la réduction des plaques d'athérome ou par la prévention de la formation des plaques d'athéromes.
L'invention concerne aussi une composition pharmaceutique comprenant au moins une méthyl-cyclodextrine présentant un degré de substitution molaire compris entre 0,4 et 0,9 pour son utilisation dans le traitement et/ou la prévention de l'athérosclérose ou des complications liées à un athérome, et/ou les maladies du système nerveux central, notamment la maladie d'Alzheimer, de Parkinson ou de Niemann Pick type C.
L'invention concerne aussi des méthodes ou des procédés mettant en œuvre une composition pharmaceutique comprenant au moins une méthyl-cyclodextrine telle que définie dans la présente invention pour son administration dans une quantité thérapeutique efficace chez un individu souffrant d'une maladie susceptible d'être traitée et/ou prévenue par une augmentation du taux de cholestérol-HDL et/ou par une réduction ou prévention des plaques d'athérome, en particulier l'athérosclérose ou des complications liées à un athérome, et/ou les maladies du système nerveux central, notamment la maladie d'Alzheimer, de Parkinson ou de Niemann Pick type C.
L'invention concerne également l'utilisation d'une composition pharmaceutique comprenant au moins une méthyl-cyclodextrine telle que définie dans la présente demande pour la préparation d'un médicament pour traiter et/ou prévenir des maladies susceptibles d'être traitées et/ou prévenues par une augmentation de taux de cholestérol-HDL et/ou par une réduction ou prévention des plaques d'athérome, de préférence l'athérosclérose ou des complications liées à un athérome, et/ou les maladies du système nerveux central, notamment la maladie d'Alzheimer, de Parkinson ou de Niemann Pick type C.

### Les méthyl-cyclodextrines

Les cyclodextrines sont des oligosaccharides cycliques provenant de la dégradation enzymatique de l'amidon. Les trois cyclodextrines naturelles les plus courantes se composent de 6, 7 ou 8 unités α-D-glucopyranose en configuration chaise reliées entre elles par des liaisons α-1,4. On les appelle plus couramment α, β, ou γ cyclodextrine, respectivement. Leur structure en trois dimensions apparaît sous la forme d'un cône tronqué à l'extérieur duquel se trouvent les groupements hydroxyles représentant la partie hautement hydrophile des cyclodextrines. L'intérieur du cône ou la cavité des cyclodextrines est constitué par les atomes d'hydrogène portés par les carbones C₃ et C₅ ainsi que par les atomes d'oxygène participant à la liaison glycosidique, leur conférant ainsi un caractère apolaire. Les cyclodextrines présentant une partie extérieure hydrophile et une cavité hydrophobe sont généralement utilisées pour leur capacité à encapsuler les composés hydrophobes et, donc, pour leur rôle de protecteur et de solubilisant de substances actives hydrophobes. On les retrouve ainsi classiquement dans les domaines de l'agroalimentaire, mais aussi en galénique où elles sont utilisées comme excipient dans des formulations pharmaceutiques administrées par voie orale ou dans des formulations cosmétiques administrées par voie topique.

En vue d'améliorer la solubilité aqueuse des cyclodextrines naturelles, de nombreux dérivés ont été synthétisés par greffage de différents groupements sur les fonctions hydroxyles. Les unités glucopyranoses des cyclodextrines comprennent en effet chacune 3 groupements hydroxyles réactifs, qui sont portés par les carbones C2, C3 et C6.
On peut citer comme exemples de dérivés les hydroxypropyl-cyclodextrines, les méthyl-cyclodextrines et les dérivés « sulfatés » de cyclodextrine. Frömming et Szetli ont notamment montré dans le cas de méthyl-cyclodextrines que l'augmentation du degré de méthylation favorisait la solubilisation jusqu'à un degré de substitution molaire égal à 2, et qu'au-delà la solubilisation diminuait.

Certains auteurs se sont également intéressés aux cyclodextrines et à leurs dérivés autrement que par leur rôle d'excipient pharmaceutique.

Par exemple, la demande de brevet WO 02/43742 décrit le rôle de cyclodextrines ou d'hydroxypropyl-cyclodextrines dans le métabolisme du cholestérol, et notamment pour favoriser l'efflux du cholestérol des macrophages. Dans cette demande, les auteurs rappellent qu'il existe deux mécanismes d'actions des cyclodextrines. Les cyclodextrines de forte affinité pour le cholestérol agissent directement en complexant le cholestérol membranaire des macrophages. En revanche, les cyclodextrines de faible affinité pour le cholestérol agissent comme catalyseur de l'efflux du cholestérol, de la membrane cellulaire vers un accepteur extracellulaire qui est une vésicule de phospholipides. Dans ce dernier cas, l'efflux du cholestérol ne peut donc se faire qu'en présence d'un autre élément qui est une vésicule de phospholipides acceptrice exogène. Ainsi, si des effets pharmacologiques sur le cholestérol sont évoqués dans l'art antérieur, l'intérêt thérapeutique des cyclodextrines demeure fort contestable.

En effet, les cyclodextrines de forte affinité, de par leur activité dans la complexation du cholestérol, auraient nécessité d'être administrées dans des quantités considérables, qui n'auraient pas été pharmaceutiquement acceptables et qui auraient engendré des problèmes de toxicité du fait de l'importante quantité de cholestérol solubilisée.

Quant aux cyclodextrines de faible affinité, la nécessité de les co-administrer avec des vésicules de phospholipides en fait une technologie bien trop complexe au vu des connaissances actuelles, notamment parce que les vésicules de phospholipides sont très instables.

L'utilisation des méthyl-cyclodextrines parmi d'autres agents actifs a été suggérée dans le traitement de l'athérosclérose (WO 2006/032905). Notamment, cette demande de brevet suggère que les méthyl-cyclodextrines sont capable d'inhiber les liaisons entre les LDL oxydées et les monocytes. L'utilisation des méthyl-β-cyclodextrines a été suggérée dans des méthodes pour moduler la réponse inflammatoire en altérant les niveaux plasmatiques de cholestérol (US 2008/0032925). La méthyl-β-cyclodextrine utilisée dans cette demande est une méthyl-β-cyclodextrine commercialisée par Sigma Aldrich et qui présente un degré de substitution molaire bien supérieur à 1,5.

Dans la présente invention, les inventeurs utilisent des compositions de cyclodextrines particulières agissant comme principe actif dans le but d'augmenter significativement le taux de cholestérol-HDL plasmatique tout en réduisant au maximum les inconvénients liés à la toxicité chez un patient. Les compositions de l'invention permettent d'écarter les problèmes de toxicité grâce à leur caractéristique particulière, à savoir, un degré de substitution, en particulier de méthylation, relativement faible ainsi qu'une faible affinité pour le cholestérol.
De façon surprenante, et en dépit de leur faible affinité pour le cholestérol, les méthyl-cyclodextrines, et en particulier les méthyl-β-cyclodextrines, utilisées conformément à la présente invention, ne nécessitent pas l'addition de vésicules de phospholipides pour exercer leur effet.
Les résultats obtenus par les inventeurs suggèrent un mode d'action différent pour ces méthyl-cyclodextrines particulières, qui ne passerait ni par la complexation du cholestérol, ni par la catalyse d'un transport vers des vésicules de phospholipides.
De façon tout à fait originale, ces méthyl-cyclodextrines semblent promouvoir l'efflux du cholestérol sous la forme de cholestérol-HDL, et non pas sous une forme complexée à des cyclodextrines, ou à des vésicules de phospholipides.
C'est pourquoi les compositions selon la présente invention sont exemptes ou substantiellement exemptes de vésicules de phospholipides. « Subtantiellement exemptes » signifie ici que les compositions comprennent moins de 10 % de phospholipides en poids par rapport à la composition, de préférence moins de 5 % en poids et de manière particulièrement préférée, moins de 2, 1 ou 0,5 % en poids. « Subtantiellement exemptes » peut également être défini par un rapport en concentration molaire entre la quantité de cyclodextrine et celle de phospholipides qui est supérieur à 100, 1 000, ou 10 000.
La présente invention concerne donc une nouvelle utilisation d'une composition pharmaceutique comprenant au moins une méthyl-cyclodextrine présentant un degré de substitution molaire compris entre 0,4 et 0,9 dans le traitement et/ou la prévention de maladies susceptibles d'être traitées et/ou prévenues par une augmentation du taux de cholestérol-HDL et/ou par une réduction ou prévention des plaques d'athérome.

L'invention concerne aussi une nouvelle utilisation d'une composition pharmaceutique comprenant au moins une méthyl-cyclodextrine présentant un degré de substitution molaire compris entre 0,4 et 0,9 dans le traitement et/ou la prévention de l'athérosclérose ou des complications liées à un athérome, ou les maladies du système nerveux central, en particulier la maladie d'Alzheimer, de Parkinson ou de Niemann Pick de type C. On entend par « degré de substitution molaire (MS)» le nombre d'hydroxyles substitués, notamment par un groupement méthyle, par unité glucopyranose. A noter que le degré de substitution molaire (MS) est différent du degré de substitution moléculaire (DS) qui correspond au nombre d'hydroxyles substitués, notamment par un groupement méthyle, par molécule de cyclodextrine et qui tient donc compte du nombre d'unités glucopyranoses constituant la méthyl-cyclodextrine.

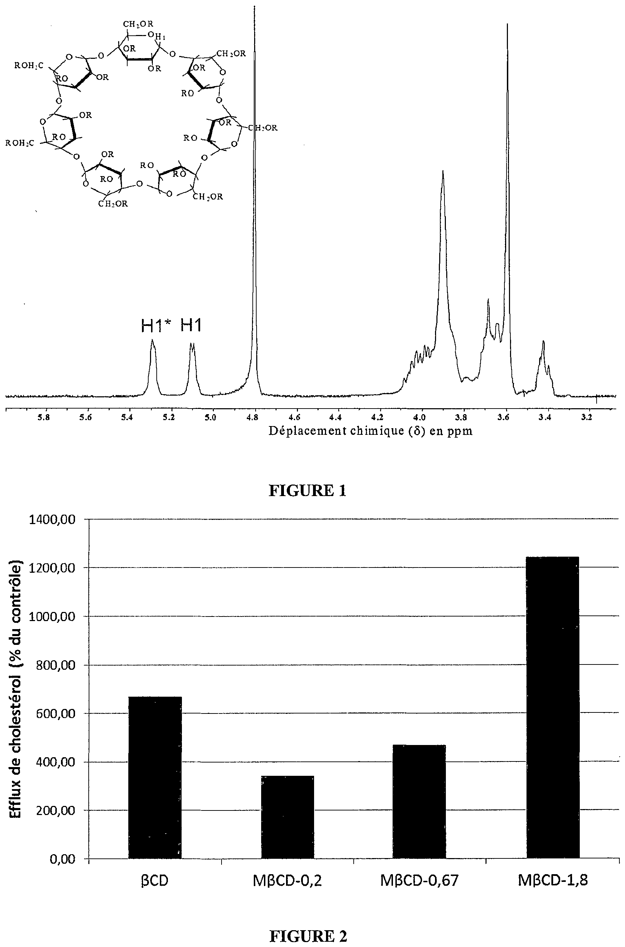

Le MS peut être déterminé dans la présente invention par Résonnance Magnétique Nucléaire du proton (RMN), ou par spectrométrie de masse (spectrométrie de masse par ionisation par électronébulisation (ESI-MS) ou spectrométrie de masse par désorption/ionisation par laser assisté par matrice (MALDI-MS)). Bien que ces techniques soient bien connues de l'homme du métier, des conditions optimales de détermination du MS des méthyl-cyclodextrines selon l'invention sont en particulier bien décrites dans la thèse de référence de JACQUET Romain. « Cyclodextrines hydrophiles : caractérisation et étude de leurs propriétés énantiosélective et complexante. Utilisation de la chromatographie en phase liquide et de la spectrométrie de masse ». Thèse de Chimie et physicochimie des composés d'intérêt biologique. Université d'Orléans, 2006. notamment disponible sur : http://tel.archives-ouvertes.fr/docs/00/18/55/42/PDF/jacquet.pdf (consulté le 27.11.2013). », en particulier Chapitre 2, Partie B (pages 59 à 83).
De préférence, le MS est déterminé par RMN, selon la méthode suivante : les mesures sont conduites à 25°C sur un appareil de type DPX 250 MHz Advance (Bruker, Rheinstetten, Allemagne). La calibration est effectuée avec le signal D₂O. Les échantillons de méthyl-cyclodextrine conforme à l'invention, et de cyclodextrine native, c'est-à-dire non méthylée, sont préparés à une concentration de 5 mg dans 0,75 mL de D₂O. Les solutions sont évaporées à sec sous courant d'azote puis reconstituées dans 0,75 mL de D₂O. Cette opération est répétée deux fois afin d'assurer un échange total des protons des fonctions hydroxyle. Le MS est calculé à partir de la différence d'intégration entre le spectre de la cyclodextrine native et celui de la méthyl-cyclodextrine conforme à l'invention. Un spectre typique est montré dans la Figure 1.

Il est à noter que la méthyl-cyclodextrine utilisée conformément à l'invention, bien que pouvant correspondre à un produit pur, correspond généralement à un mélange de méthyl-cyclodextrines de structures différentes. C'est le cas par exemple du produit commercialisé par la Demanderesse sous la dénomination KLEPTOSE ® CRYSMEB, qui présente notamment les propriétés physico-chimiques telles que déterminées dans la thèse de JACQUET Romain précitée, en particulier au chapitre 2, partie B (pages 59 à 83).
Il en résulte que le MS mesuré est dans ce cas une moyenne des substitutions qui s'opèrent sur l'ensemble des unités glucopyranoses de l'ensemble du mélange de méthyl-cyclodextrines.
Ce mélange peut notamment contenir de la cyclodextrine native résiduelle, c'est-à-dire non méthylée, mais qui se trouvent généralement en quantités négligeables, en particulier inférieures à 1% en poids sec par rapport au poids sec total de la méthyl-cyclodextrine, préférentiellement inférieur à 0,5%, préférentiellement encore inférieur à 0,1%.
Dans le contexte de l'invention, les compositions comprennent au moins une méthyl-cyclodextrine présentant un degré de substitution molaire compris préférentiellement entre 0,5 et 0,9, préférentiellement entre 0,6 et 0,8, par exemple 0,7, notamment 0,67. Par exemple, la méthyl-cyclodextrine peut avoir un MS compris entre 0,50 et 0,90, entre 0,60 et 0,80.

Préférentiellement, au moins 50% des groupements méthyles de la méthyl-cyclodextrine utilisée dans le cadre de la présente invention sont situés au niveau de l'hydroxyle porté par le carbone C2 de l'unité glucopyranose, préférentiellement entre 60 et 80%, typiquement de l'ordre de 75%.
Parallèlement, les autres groupements méthyles sont généralement majoritairement situés au niveau de l'hydroxyle porté par le carbone C3 et/ou C6 de l'unité glucopyranose.

L'homme du métier sait comment déterminer la répartition des groupements méthyles sur les hydroxyles de l'unité glucopyranose de la méthyl-cyclodextrine par exemple par RMN. Avantageusement, la méthyl-cyclodextrine utilisée dans le cadre de la présente invention comporte 7 unités α-D-glucopyranose. Il s'agit donc d'une méthyl-β-cyclodextrine.
Dans un mode de réalisation particulier, la méthyl-cyclodextrine est une méthyl-β-cyclodextrine et a un MS compris préférentiellement entre 0,5 et 0,9, préférentiellement entre 0,6 et 0,8, par exemple 0,7, notamment 0,67. Par exemple, la méthyl-cyclodextrine peut avoir un MS compris entre 0,50 et 0,90, entre 0,60 et 0,80.
La méthyl-cyclodextrine peut être substituée sur l'hydroxyle porté par le carbone C2 des unités glucopyranoses, ou par les carbones C3 et/ou C6 des unités glucopyranoses, ou par une combinaison des carbones C2, C3 et/ou C6, de préférence C2 et C6 des unités glucopyranoses.
Dans un autre mode de réalisation particulier, la méthyl-cyclodextrine est une méthyl-cyclodextrine, de préférence une méthyl-β-cyclodextrine, dont au moins 50% des groupements méthyle sont situés au niveau de l'hydroxyle porté par le carbone C2 de l'unité glucopyranose, préférentiellement entre 60 et 80%, typiquement de l'ordre de 75%, et a un MS compris préférentiellement entre 0,5 et 0,9, préférentiellement entre 0,6 et 0,8, par exemple 0,7, notamment 0,67. Par exemple, la méthyl-cyclodextrine peut avoir un MS compris 0,50 et 0,90, entre 0,60 et 0,80.
Dans un mode de réalisation préféré, la composition de méthyl-cyclodextrines comprend une ou plusieurs méthyl- β-cyclodextrines choisies parmi le groupe consistant en des méthyl-β-cyclodextrines substituées sur l'hydroxyle porté par le carbone C2 des unités glucopyranoses, des méthyl-β-cyclodextrines substituées sur l'hydroxyle porté par le carbone C3 et/ou C6 des unités glucopyranoses, des méthyl-β-cyclodextrines substituées sur l'hydroxyle porté par les carbones C2, C3 et/ou C6, de préférence C2 et C6 des unités glucopyranoses et ayant un MS compris préférentiellement entre 0,5 et 0,9, préférentiellement entre 0,6 et 0,8, par exemple 0,7, notamment 0,67. Par exemple, la méthyl-cyclodextrine peut avoir un MS compris entre 0,50 et 0,90, entre 0,60 et 0,80. De préférence, la composition de méthyl-cyclodextrines comprend au moins 50, 60, ou 75 % de méthyles substituées sur l'hydroxyle porté par le carbone C2 des unités glucopyranoses.
Comme évoqué précédemment, la méthyl-cyclodextrine selon l'invention peut être un mélange. L'analyse par spectrométrie de masse du produit KLEPTOSE® CRYSMEB, qui est une méthyl-β-cyclodextrine, révèle en particulier qu'il s'agit d'un produit polydispersé, comprenant sept groupes de méthyl-cyclodextrines majoritaires, qui se distinguent par leur DS. Ce DS, qui en théorie peut varier de 0 à 21 pour une méthyl-β-cyclodextrine, varie de 2 à 8 dans le produit KLEPTOSE® CRYSMEB.

Avantageusement, les compositions de l'invention comprennent un mélange de méthyl-cyclodextrines comprenant au moins 50, 60, 70, 80 ou 90% de méthyl-cyclodextrines présentant un MS compris entre 0,2 et 1,2. De préférence au moins 40, 50, 60, 70, 80 ou 90% de méthyl-cyclodextrines présentent un MS compris entre 0,3 et 1,1. De préférence au moins 30, 40, 50, 60, 70, 80 ou 90% de méthyl-cyclodextrines présentent un MS compris entre 0,5 et 0,9. Encore plus préférentiellement, au moins 25, 30, 40, 50, 60, 70, 80 ou 90% de méthyl-cyclodextrines présentent un MS compris entre 0,6 et 0,8.
Les compositions de méthyl-cyclodextrines peuvent être éventuellement préparées en ajoutant différentes méthyl-cyclodextrines présentant des MS définis pour obtenir des compositions telles que définies dans la présente invention ou elles peuvent être obtenues comme résultat de la synthèse de celles-ci.

Ainsi dans un autre mode de réalisation particulier, la composition de méthyl-cyclodextrines, de préférence de méthyl-β-cyclodextrines, présente le profil de substitution, exprimée en pourcentages molaires, suivant :
∘ 0 à 5 % de méthyl-β-cyclodextrines comprennent 2 groupements méthyles (DS de 2) ;
∘ 5 à 15 % de méthyl-β-cyclodextrines comprennent 3 groupements méthyles (DS de 3) ;
∘ 20 à 25 % de méthyl-β-cyclodextrines comprennent 4 groupements méthyles (DS de 4) ;
∘ 25 à 40 % de méthyl-β-cyclodextrines comprennent 5 groupements méthyles (DS de 5) ;
∘ 15 à 25 % de méthyl-β-cyclodextrines comprennent 6 groupements méthyles (DS de 6) ;
∘ 5 à 15 % de méthyl-β-cyclodextrines comprennent 7 groupements méthyles (DS de 7) ;
∘ 0 à 5 % de méLhyl-β-cyclodexLrines comprennent 8 groupements méthyles (DS de 8) ;
la somme totale étant généralement de l'ordre de 100%, bien que la composition puisse contenir éventuellement des traces de méthyl-cyclodextrines de DS différent, ainsi que des traces de cyclodextrine native, c'est-à-dire non méthylée.

Le profil de substitution peut être déterminé par toute technique bien connue de l'homme du métier, par exemple par ESI-SM ou MALDI-TOF-SM. Les conditions optimales de détermination du profil de substitution par ces deux méthodes sont notamment largement discutées dans la thèse de Romain JACQUET précitée, au chapitre 2, partie B, points II.3 et II.2 (page 67 à 82) et à l'Annexe II.

Dans un mode de réalisation préféré, la composition de méthyl-cyclodextrines, de préférence de méthyl-β-cyclodextrines, est telle qu'au moins 50% des groupements méthyles sont situés au niveau de l'hydroxyle porté par le carbone C2 des unités glucopyranoses, préférentiellement entre 60 et 80%, typiquement de l'ordre de 75%, et qui présente le profil de substitution, exprimé en pourcentages molaires, suivant :
∘ 0 à 5 % de méthyl-β-cyclodextrines comprennent 2 groupements méthyles (DS de 2) ;
∘ 5 à 15 % de méthyl-β-cyclodextrines comprennent 3 groupements méthyles (DS de 3) ;
∘ 20 à 25 % de méthyl-β-cyclodextrines comprennent 4 groupements méthyles (DS de 4) ;
∘ 25 à 40 % de méthyl-β-cyclodextrines comprennent 5 groupements méthyles (DS de 5) ;
∘ 15 à 25 % de méthyl-β-cyclodextrines comprennent 6 groupements méthyles (DS de 6) ;
∘ 5 à 15 % de méthyl-β-cyclodextrines comprennent 7 groupements méthyles (DS de 7) ;
∘ 0 à 5 % de méthyl-β-cyclodextrines comprennent 8 groupements méthyles (DS de 8) ;
la somme totale étant généralement de l'ordre de 100%, bien que la composition puisse contenir éventuellement des traces de méthyl-cyclodextrines de DS différent, ainsi que des traces de cyclodextrine native, c'est-à-dire non méthylée.

Il est par ailleurs tout à fait possible d'envisager de faire varier en proportions ou d'isoler des molécules ou groupes de molécules de méthyl-cyclodextrines, notamment en fonction de leur DS.

Ainsi, dans un autre mode de réalisation particulier, la méthyl-cyclodextrine est une méthyl-β-cyclodextrine qui présente un DS choisi parmi un nombre entier allant de 2 à 8, en particulier 2, 3, 4, 5, 6, 7 ou 8.

Dans un autre mode de réalisation préféré, la méthyl-cyclodextrine est une méthyl-β-cyclodextrine dont au moins 50% des groupements méthyles sont situés au niveau de l'hydroxyle porté par le carbone C2 des unités glucopyranoses, préférentiellement entre 60 et 80%, typiquement de l'ordre de 75%, et qui présente un DS choisi parmi un nombre entier allant de 2 à 8, en particulier 2, 3, 4, 5, 6, 7 ou 8.

Dans un autre mode de réalisation particulier, la méthyl-cyclodextrine, en particulier la méthyl-β-cyclodextrine, a un MS compris entre 0,5 et 0,6 notamment entre 0,50 et 0,60. Dans un autre mode de réalisation particulier, la méthyl-cyclodextrine, en particulier la méthyl-β-cyclodextrine, a un MS compris entre 0,6 et 0,7 notamment entre 0,60 et 0,70. Dans un autre mode de réalisation particulier, la méthyl-cyclodextrine, en particulier la méthyl-β-cyclodextrine, a un MS compris entre 0,7 et 0,8 notamment entre 0,70 et 0,80. Dans un autre mode de réalisation particulier, la méthyl-cyclodextrine, en particulier la méthyl-β-cyclodextrine, a un MS compris entre 0,8 et 0,9 notamment entre 0,80 et 0,90.

Généralement, la méthyl-cyclodextrine utilisée conformément à l'invention présente un taux de sucres réducteurs inférieur à 1% en poids sec, préférentiellement inférieur à 0,5%.

La composition de méthyl-β-cyclodextrines selon l'invention peut être obtenue par le procédé décrit dans le brevet US 6,602,860 B1. Un exemple d'une telle composition est commercialisé par le groupe ROQUETTE FRERES sous la dénomination commerciale KLEPTOSE® CRYSMEB et présente un degré de substitution molaire de 0,7 ou plus précisément de 0,67 méthyles par unité de glucose.
Facultativement, la composition selon la présente invention peut comprendre en outre une cyclodextrine, en particulier β-cyclodextrine, non substituée et/ou une cyclodextrine, en particulier β-cyclodextrine, substituée par des groupes sulfobutyléther (SBE-), hydroxyéthyles, hydroxypropyles (HP-), carboxyméthyles, carboxyéthyles, acétyles, triacétyles, succinyles, éthyles, propyles, butyles, sulfates, de préférence sulfobutyles et hydroxypropyles, de préférence avec un degré de substitution molaire compris entre 0,05 et 1,5.

Facultativement, la méthyl-cyclodextrine selon l'invention, en particulier la méthyl-β-cyclodextrine, peut être substituée par des groupes additionnels, notamment choisis parmi ceux énumérés avant. Il pourra donc par exemple s'agir d'une méthyl- β-cyclodextrine sulfatée.

Dans un mode de réalisation distinct, la présente invention considère également l'utilisation pour le traitement des maladies selon la présente invention d'autres dérivés de cyclodextrines, de préférence de β-cyclodextrine, présentant un degré de substitution molaire conforme à la présente invention, c'est-à-dire compris préférentiellement entre 0,5 et 0,9, préférentiellement entre 0,6 et 0,8, par exemple 0,7. Ces dérivés de cyclodextrine, de préférence de β-cyclodextrine, sont substitués par un groupe choisi parmi les sulfobutyléthers (SBE-) et les hydroxypropyles (HP-). De préférence, ces substitutions sont majoritairement portées par le carbone C2 des unités glucopyranoses, typiquement à 50 %, 60 %, 70% ou 80 %.
Dans un mode alternatif de l'invention, les méthyl-cyclodextrines telles que définies dans la présente demande et composées d'unités α-D-glucopyranoses reliées entre elles par des liaisons α-1,4 peuvent être substituées en partie ou totalité par des unités α-D-glucopyranoses reliées entre elles par des liaisons α-1,6, dans les compositions pharmaceutiques de la présente invention.

### Application

Dans le contexte de la présente invention, les compositions pharmaceutiques comprenant au moins une méthyl-cyclodextrine telle que définie dans la présente demande sont utilisées dans le traitement et/ou la prévention de maladies susceptibles d'être traitées et/ou prévenues par une augmentation du taux de cholestérol-HDL et/ou par une réduction ou prévention des plaques d'athérome, en particulier de l'athérosclérose ou des complications liées à un athérome, et/ou les maladies du système nerveux central, en particulier la maladie d'Alzheimer, de Parkinson ou de Niemann Pick de type C.

Dans le contexte de la présente invention, l'augmentation du taux de cholestérol-HDL est mesurée dans tout type de fluide biologique. Il s'agit préférentiellement du taux de cholestérol-HDL plasmatique. Ce taux de cholestérol-HDL peut être mesuré par toute méthode connue de l'homme du métier, par exemple par précipitation, ou par des méthodes directes en phase homogène utilisant des techniques immunologiques. Un dosage de la partie protéique (protéine Apo Al) composant les HDL est également possible.

Le taux de cholestérol-HDL est généralement exprimé en nombre de moles de cholestérol transporté par les lipoprotéines de haute densité (HDL) par litre de sang. Le cholestérol-HDL est apparenté au « bon cholestérol » au contraire du cholestérol transporté par les LDL.

Selon l'invention, les maladies susceptibles d'être traitées et/ou prévenues par une augmentation du taux de cholestérol-HDL sont toutes les maladies dont les symptômes et/ou les causes disparaissent ou sont atténués chez un patient lorsque le taux de cholestérol-HDL est supérieur au taux de cholestérol-HDL avant traitement. De préférence, les maladies sont celles susceptibles d'être traitées et/ou prévenues par une augmentation du taux de cholestérol-HDL. Par « augmentation », peut être entendue une augmentation du taux de cholestérol-HDL d'au moins 5, 7,5, 10, 15 ou 20 %.

Les maladies susceptibles d'être traitées et/ou prévenues par une augmentation du taux de cholestérol-HDL sont préférentiellement les maladies liées à une surcharge, et/ou à un stockage, et/ou à l'accumulation de cholestérol dans les tissus, ainsi que leurs conséquences.

Comme exemples de maladies susceptibles d'être liées à une surcharge, et/ou à un stockage, et/ou à l'accumulation de cholestérol dans les tissus, on peut citer les maladies cardiovasculaires, les maladies vasculaires, les maladies artérielles périphériques occlusives telles que l'athérosclérose ou les complications liées à un athérome.

Comme autres exemples, on peut citer les maladies du système nerveux central susceptibles d'être liées à une surcharge, et/ou à un stockage, et/ou à l'accumulation de cholestérol dans les tissus du système nerveux central. De telles maladies comprennent de manière non limitative la maladie d'Alzheimer, la maladie de Parkinson, et les maladies lysosomale affectant le système nerveux central.

Comme exemples de maladies lysosomales, on peut citer sans limitation la maladie de Niemann-Pick, telle que la maladie de Niemann-Pick type A, la maladie de Niemann-Pick type B, ou la maladie de Niemann-Pick type C.

Un exemple particulier, est la maladie de Niemann-Pick type C caractérisée par une accumulation de cholestérol non estérifié dans le Système Nerveux Central (SNC).

Dans un mode de réalisation préféré, la maladie est sélectionnée parmi la maladie d'Alzheimer, la maladie de Parkinson et la maladie de Niemann-Pick de type C.

Un autre exemple particulier concerne la maladie d'Alzheimer. L'accumulation de cholestérol dans le cerveau peut également être à l'origine de la maladie d'Alzheimer et une augmentation du taux de cholestérol-HDL favorise son transport vers le foie où il est dégradé. Des données suggèrent de manière insistante qu'un dysfonctionnement du métabolisme du cholestérol dans le cerveau et au niveau du système vasculaire est selon toute probabilité étroitement impliqué dans la maladie d'Alzheimer.

La présence d'un taux trop élevé de cholestérol est souvent considérée comme un facteur de risque pour l'apparition de troubles cognitifs, de démence ou de maladie de Parkinson. L'implication du cholestérol dans l'augmentation du risque de maladie de Parkinson a également été évoquée dans plusieurs études.

Dans ce contexte, un traitement dont l'effet est une augmentation du taux de cholestérol-HDL qui contribue au transport du cholestérol en excès vers le foie où il est dégradé prend tout son sens pour cette application. En effet, un faisceau d'observations, de données statistiques et d'expériences au niveau moléculaire conduisent à accorder un rôle de plus en plus significatif au métabolisme du cholestérol dans la genèse de la maladie d'Alzheimer. En particulier une convergence entre l'athérosclérose et la maladie d'Alzheimer (AD) ainsi qu'entre la maladie d'Alzheimer et la maladie de Niemann-Pick type C est solidement établie. Cette dernière maladie rare est caractérisée par l'accumulation excessive de cholestérol dans les cellules nerveuses.

Par ailleurs, la présente invention concerne également les maladies susceptibles d'être traitées et/ou prévenues par une réduction ou prévention des plaques d'athérome. Par réduction des plaques d'athérome est entendu de préférence que les plaques présentent une surface plus faible en présence du traitement qu'en absence de celui-ci. Notamment, la réduction de la surface des plaques de 10, 20, 30, 40 ou 50 %. De préférence, cette réduction est d'au moins 30 ou 40 %. La surface des plaques d'athérome peut être déterminée par toute méthode connue de l'homme du métier et notamment les méthodes d'imagerie médicales telles que l'IRM (Imagerie par Résonnance Magnétique). Par prévention des plaques d'athérome, on entend un ralentissement du développement ou de la formation de ces plaques.

Les maladies susceptibles d'être traitées et/ou prévenues par une réduction ou prévention des plaques d'athérome, sont en particulier les maladies cardiovasculaires, les maladies vasculaires, et préférentiellement les maladies artérielles périphériques occlusives telles que l'athérosclérose ou les complications liées à un athérome.

Selon l'invention, les maladies susceptibles d'être traitées et/ou prévenues sont préférentiellement l'athérosclérose ou les complications liées à un athérome, la maladie d'Alzheimer, la maladie de Parkinson ou la maladie de Niemann-Pick type C et, avantageusement, l'athérosclérose ou les complications liées à un athérome.

Les complications liées à un athérome qui sont traitées et/ou prévenues par l'utilisation d'une composition pharmaceutique comprenant au moins une méthyl-cyclodextrine de l'invention sont de manière non limitative l'ischémie, par exemple l'ischémie du myocarde, les maladies coronariennes, l'angine de poitrine, le syndrome coronarien aigu, l'infarctus du myocarde, l'infarctus mésentérique, l'accident vasculaire-cérébral, l'anévrisme ou l'artériopathie des membres inférieurs.

Dans un autre mode de réalisation particulier, les compositions de l'invention utilisées comprennent en outre au moins un agent actif supplémentaire. L'agent actif supplémentaire est préférentiellement choisi dans le groupe des agents actifs connus par l'homme du métier pour traiter et/ou prévenir l'athérosclérose ou les complications liées à un athérome.

Les sujets à traiter sont de préférence des humains ou des animaux, de préférence des humains. Ils peuvent avoir une maladie avérée (diagnostiquée ou établie) ou être à risque de développer cette maladie. Notamment, la présente invention entend par « traitement » une diminution des causes ou symptômes d'une maladie, le retard dans l'apparition de la maladie, un ralentissement du développement de celle-ci mais également une guérison.

Comme exemples d'actifs utilisés dans le traitement de l'athérosclérose ou des complications liées à un athérome, on peut citer :
- les statines ;
- les agents anti-hypertenseurs, en particulier :
   ∘ les inhibiteurs de l'enzyme de conversion de l'angiotensine, par exemple le perindopril, le captopril, l'enalapril, le lisinopril, ou le ramipril ;
   ∘ les antagonistes du récepteur à l'angiotensine II, aussi connus sous le nom de « sartans », tels que le losartan, le valsartan, le candesartan ;
   ∘ les beta-bloquants, tels que l'acébutolol, le labétalol, le nadolol, l'oxprénolol, le penbutolol, le pindolol, le propranolol ;
- les antiagrégants plaquettaires ou anticoagulants, en particulier :
   ∘ l'asipirine ;
   ∘ le clopidogrel (PLAVIX®) ;
   ∘ nouveaux anticoagulants oraux tels que le dabigatran/Pradaxa®, l' apixaban/Eliquis®, le rivaroxaban/Xarelto®, indiqués dans la prévention des phlébites, des embolies ou des accidents vasculaires cérébraux dans l'indication fibrillation auriculaire ;
   ∘ anti-vitamines K, très efficaces mais peu utilisés aujourd'hui car nécessitent une surveillance très étroite de la coagulation.

De préférence, l'agent actif supplémentaire sera préférentiellement choisi parmi les antagonistes du récepteur à l'angiotensine II.

Les compositions selon la présente invention sont susceptibles d'être administrées par voie orale, parentérale, mucosale ou cutanée. La voie parentérale comprend préférentiellement l'administration sous-cutanée, intraveineuse, intramusculaire ou intrapéritonéale, bien que cette dernière soit plutôt réservée à l'animal. La voie mucosale comprend préférentiellement l'administration par voie nasale, par voie pulmonaire, par la muqueuse rectale. La voie cutanée comprend avantageusement la voie dermique, notamment via un dispositif transdermique, typiquement un patch. D'autres voies d'administration concernant plus particulièrement les maladies touchant le Système Nerveux Central (SNC), en particulier la maladie de Niemann-Pick type C, la maladie de Parkinson ou la maladie d'Alzheimer, sont la voie intrathécale ou rachidienne.

Les compositions selon la présente invention comprennent également un excipient pharmaceutiquement acceptable. On peut utiliser tout excipient adapté pour les formes galéniques connues de l'homme du métier en particulier en vue d'une administration systémique, préférentiellement en vue d'une administration orale d'une administration parentérale, d'une administration cutanée ou mucosale, notamment par voie sous-cutanée, intraveineuse, intramusculaire, intrapéritonéale, nasale, pulmonaire, rectale, dermique, intrathécale ou rachidienne.
On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables) sont notamment la méthylcellulose, l'hydroxyméthylcellulose, la carboxyméthylcellulose, le polysorbate 80, le mannitol, la gélatine, le lactose, des huiles végétales, l'acacia, les liposomes, etc. Les compositions peuvent être formulées sous forme de suspensions injectables, gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

On peut avantageusement imaginer une forme galénique dans laquelle la cyclodextrine selon l'invention est administrée sous une forme complexée à une substance lipidique, de façon à permettre son passage dans la circulation sanguine après administration par des voies non invasives, et/ou dans le cas où le passage de la barrière-hémato-encéphalique est souhaité, pour permettre le passage de cette barrière. Parmi ces substances lipidiques, on peut notamment citer le tocophérol.

Les compositions susceptibles d'être administrées chez un individu dans le cadre de l'invention comprennent entre 1 et 100 mg/kg, préférentiellement entre 20 et 70 mg/kg, encore plus préférentiellement entre 30 et 50 mg/kg, et d'une manière encore plus préférée 40 mg/kg de méthyl-cyclodextrine telle que définie dans la présente invention, par rapport au poids total de l'individu. Bien entendu, l'homme du métier est en mesure d'adapter la dose de méthyl-cyclodextrine définie dans la présente demande en fonction du poids de l'individu à traiter.

Avantageusement, les compositions sont donc susceptibles d'être administrées dans une quantité thérapeutique efficace chez un individu souffrant d'une maladie susceptible d'être traitée et/ou prévenue par une augmentation du taux de cholestérol-HDL, ou chez un individu prédisposé à développer une telle maladie. On entend par quantité thérapeutique efficace, une quantité de composition suffisante susceptible d'être administrée chez un individu pour prévenir et/ou traiter une maladie telle que définie dans la présente invention.

Les compositions utilisées dans l'invention peuvent être administrées selon des modalités variables. En particulier, elles peuvent être injectées une à cinq fois par semaines pendant 1, 2, 3, 4 semaines, voire 1, 2, 3 ou plusieurs mois. Les modalités d'administration incluent également les traitements à intervalles espacés de plusieurs semaines ou de plusieurs mois. Pour le traitement et/ou la prévention de l'athérosclérose en particulier, on envisagera avantageusement une administration à intervalle de 3 à 6 mois, pouvant aller jusqu'à 12 mois, d'une durée d'une semaine.

Un autre objet de l'invention concerne l'utilisation des compositions pharmaceutiques telles que définies dans la présente demande pour favoriser en outre une diminution du taux de triglycérides et d'acides gras circulants.

Un objet supplémentaire de l'invention concerne l'utilisation des compositions pharmaceutiques telles que définies dans la présente demande pour réduire les plaques d'athérome.

L'utilisation des compositions de l'invention permettent à la fois d'augmenter le taux de cholestérol-HDL, de diminuer les taux de triglycérides et d'acides gras circulant dans le sang et de réduire les plaques d'athérome. La composition de l'invention permet également d'induire une diminution des MMP-9 responsables de la fragilisation des plaques d'athérome, et donc des complications liées à l'athérome. Tous ces effets sont obtenus sans modification du taux de cholestérol-LDL, et sans qu'aucune toxicité n'ait été relevée. La diminution des MMP-9 suggère par ailleurs l'intérêt de la composition selon l'invention pour une utilisation dans le traitement et/ou la prévention du cancer.

Les compositions telles que définies dans la présente demande présentent également un intérêt chez les sujets sains. Les compositions de l'invention permettent notamment de réduire le taux de triglycérides et la concentration d'acides gras libres et d'augmenter le taux de bon cholestérol (Cholestérol-HDL) favorisant ainsi la bonne santé du sujet. C'est pourquoi la présente invention est également relative à une utilisation non-thérapeutique des compositions de méthyl-cyclodextrines telles que définies précédemment dans la présente demande pour réduire le taux de triglycérides et/ou pour réduire la concentration d'acides gras libres et/ou pour augmenter le taux de bon cholestérol (Cholestérol-HDL). Ainsi, la composition de méthyl-cyclodextrines selon la présente invention peut être utilisée en tant que composition nutraceutique, alicament, aliment fonctionnel ou complément alimentaire. Par composition nutraceutique, alicament, aliment fonctionnel, on entend ici un aliment qui contient des ingrédients ayant des effets bénéfiques pour la santé ou capables d'améliorer les fonctions physiologiques. Par complément alimentaire, on entend une denrée ayant pour but de compléter le régime alimentaire normal.
Les sujets normaux ou sains les plus propices à tirer bénéfice de ces compositions sont des individus ayant 40 ans ou plus, de préférence 50 ans ou plus, et par exemple ayant entre 50 et 70 ans. Ces sujets ne souffrent d'aucune pathologie.
La composition est destinée à l'alimentation humaine peut prendre toute forme, et notamment être un liquide, une pâte ou un solide. Notamment, la composition peut être un produit laitier tel que du fromage, du beurre, un yaourt ou une crème, un produit à base de fruit comme un jus de fruit, une compote ou une pâte de fruit, une boisson, ou un aliment solide, comme un snack, un biscuit, ou autre. Elle peut également être formulée sous forme de comprimés, d'une poudre, d'une capsule, d'un comprimé, d'une solution, d'un concentré, d'un sirop, d'une suspension, ou d'une dispersion.

Les exemples qui suivent servent à illustrer et montrer d'autres aspects et avantages de l'invention et doivent être considérés non limitatifs.

### LEGENDE DES FIGURES

**Figure 1** **:** Spectre RMN¹H d'une méthyl-β-cyclodextrine selon l'invention.
**Figure 2** **:** Mesure des efflux de cholestérol en fonction du MS des méthyl-β-cyclodextrines.
**Figure 3** **:** Expression du transporteur ABCA1 en fonction du MS des méthyl-β-cyclodextrines.
**Figure 4** **:** Expression du transporteur ABCG1 en fonction du MS des méthyl-β-cyclodextrines.
**Figure 5** **:** Expression du récepteur SR-B1 en fonction MS des méthyl-β-cyclodextrines.

### EXEMPLES

### Exemple 1 : Etude in vivo des méthyl-cyclodextrines de l'invention.

Des souris mâles Apo E -/- âgées de 11 semaines ont été utilisées.
La composition de méthyl-cyclodextrine utilisée est le produit commercial KLEPTOSE® CRYSMEB (ROQUETTE).
Les souris ont été distribuées en 6 groupes :
- 2 groupes de souris soumises à un régime normal, durant 16 semaines. Ce modèle d'athérome est caractérisé par une maladie précoce. le premier groupe a été traité avec une solution placébo (souris témoins, n=9), le second groupe a été traité avec 40 mg/kg KLEPTOSE® CRYSMEB (souris traitées, n = 12).
- 2 groupes de souris soumises à un régime riche en cholestérol, durant 11 semaines. Ce modèle d'athérome est caractérisé par une maladie avancée. le premier groupe a été traité avec une solution placébo (souris témoins, n=9), le second groupe a été traité avec 40 mg/kg KLEPTOSE® CRYSMEB (souris traitées, n = 11).

Les traitements ont été effectués par injection intrapéritonéale d'un volume de 200 µL de placébo (solution de PBS) ou de solution de KLEPTOSE® CRYSMEB, trois fois par semaine.
Les prélèvements sanguins ont été effectués au sacrifice des animaux, après les 16 ou 11 semaines de traitement, par ponction cardiaque, afin de déterminer l'impact du traitement sur la lipidémie.
D'autres valeurs telles la glycémie, les valeurs d'hématocrite (taux d'hémoglobine, numération des érythrocytes, leucocytes, lymphocytes et plaquettes) ont été mesurées sans qu'aucune différence n'ait été observée entre les différents groupes de souris. Par conséquent, ces résultats ne sont pas présentés ici.
Après sacrifice, une quantification de la surface athéromateuse a par ailleurs été réalisée sur chaque groupe de souris par « computed-assisted morphometry », sur 4 sections aortiques de 10 µm marquées à l'hématoxiline et Oil-ref-O, prélevées à 200, 400, 600 et 800 µm après le départ de la valve cuspide, selon la méthode décrite dans l'article
« Caligiuri G, Groyer E, Khallou-Laschet J, Al Haj Zen A, Sainz J, Urbain D et al. Reduced immunoregulatory CD31+ T cells in the blood of atherosclerotic mice with plaque thrombosis. Arterioscler Thromb Vasc Biol 2005;25:1659-1664. ». Des marquages immuno-histochimiques des MMP-9 des plaques d'athérome ont également été effectués et quantifiés afin de déterminer leur fragilité (kit R&D Systems).
Les résultats obtenus sont présentés dans les Tableaux 1 et 2.

**Tableau 1 : Souris soumises à un régime normal et traitées avec une composition de méthyl-cyclodextrines selon l'invention**

| | **Souris témoins (n=9)** | **Souris traitées (n=12)** | ***p*** |
|---|---|---|---|
| Poids du corps (g) | 31,84 ± 2,70 | 30,71 ± 3,09 | 0,4137 |
| Lipidémie | | | |
| Cholestérol total (mmol/L) | 14,49 ± 1,65 | 14,19 ± 3,02 | 0,8070 |
| Cholestérol-LDL (mmol/L) | 9,29 ± 1,22 | 9,76 ± 2,34 | 0,8621 |
| **Cholestérol-HDL (mmol/L)** | **2,77 ± 0,15** | **3,03 ± 0,38** | **0,1451** |
| **Triglycérides (mmol/L)** | **2,20 ± 0,66** | **1,65 ± 0,55** | **0,0491** |
| **Acides gras libres (mmol/L)** | **1,33 ± 0,36** | **1,03 ± 0,40** | **0,0955** |

| **Sinus de la valve aortique** | | | |
|---|---|---|---|
| **Oil-red-O, x 10³ µm²** | **295,06 ± 36,25** | **246,14 ± 46,38** | **0,0278** |
| **MMP-9, %** | **6,35 ± 4,78** | **3,26 ± 4,84** | **0,0481** |

| **Aorte** | | | |
|---|---|---|---|
| **Surface de la plaque (%)** | **5,56 ± 2,65** | **3,39 ± 1,26** | **0,0278** |

| | | | |
|---|---|---|---|
| Les valeurs sont exprimées en moyenne ± écart-type. Les valeurs de *p* sont calculées selon le test U Mann-Whitney. | | | |

**Tableau 2 : Souris soumises à un régime riche en cholestérol et traitées avec une composition de méthyl-cyclodextrines selon l'invention**

| | **Souris témoins (n=9)** | **Souris traitées (n=11)** | ***p*** |
|---|---|---|---|
| Poids du corps (g) Lipidémie | 30,44 ± 2,34 | 28,34 ± 2,80 | 0,1194 |
| Cholestérol total (mmol/L) | 21,48 ± 1,37 | 20,17 ± 1,37 | 0,4119 |
| Cholestérol-LDL (mmol/L) | 17,05 ± 3,81 | 17,51 ± 4,00 | 0,9999 |
| **Cholestérol-HDL (mmol/L)** | **3,44 ± 0,49** | **4,16 ± 0,82** | **0,0334** |
| **Triglycérides (mmol/L)** | **1,47 ± 0,65** | **0,90 ± 0,36** | **0,0136** |
| **Acides gras libres (mmol/L)** | **0,99 ± 0,25** | **0,75 ± 0,20** | **0,0310** |

| **Sinus de la valve aortique** | | | |
|---|---|---|---|
| Oil-red-O, x 10³ µm² | 465,56 ± 66,99 | 439,45 ± 86,58 | 0,4119 |
| **MMP-9, %** | **22,80 ± 15,88** | **12,45 ± 9,80** | **0,1119** |

| **Aorte** | | | |
|---|---|---|---|
| **Surface de la plaque (%)** | **24,73 ± 6,75** | **15,00 ± 7,56** | **0,0200** |

| | | | |
|---|---|---|---|
| Les valeurs sont exprimées en moyenne ± écart-type. Les valeurs de *p* sont calculées selon le test U Mann-Whitney. | | | |

Il faut relever dans ces résultats que le déplacement des valeurs résultant du traitement s'obtient non seulement après un régime riche, mais également et de façon surprenante et favorable à la suite d'un régime normal, non enrichi en cholestérol.

Dans les deux cas en effet, une augmentation du taux de cholestérol-HDL a été observée, montrant l'intérêt des méthyl-cyclodextrines selon l'invention pour une utilisation dans le traitement et/ou la prévention de maladies susceptibles d'être traitées et/ou prévenues par une augmentation du taux de cholestérol-HDL.

Dans les deux cas, la surface des plaques d'athérome est significativement diminuée. Une diminution sensible, de près de 40%, de la surface athéromateuse a été observée. Ces résultats démontrent l'intérêt des méthyl-cyclodextrines selon l'invention, pour une utilisation dans le traitement et/ou la prévention de l'athérosclérose ou des complications liées à un athérome,

Une diminution du taux de triglycérides, des acides gras libres et du taux de MMP-9 ont été observées, et renforcent l'intérêt des méthyl-cyclodextrines selon l'invention, pour une utilisation dans le traitement et/ou la prévention de l'athérosclérose ou des complications liées à un athérome.

### Exemple 2 : Etude in vitro des méthyl-cyclodextrines de l'invention.

Cet exemple illustre l'effet de différentes cyclodextrines sur les efflux de cholestérol testés avec des cellules endothéliales aortiques. L'endothélium aortique est le tissu de la plaque d'athérome qui se trouve en contact direct avec le compartiment sanguin, et donc en contact direct avec les HDL.

### Matériel

**Cellules** : les cellules endothéliales étaient des cellules endothéliales aortiques de bovin adulte (ABAE).

**Cyclodextrines** : les cyclodextrines utilisées étaient les suivantes : une β-cyclodextrine non méthylée, ci-après désignée « β-CD », une méthyle-β-cyclodextrine présentant un MS de 0,2, ci-après désignée « MβCD-0,2 », une méthyle-β-cyclodextrine présentant un MS de 0,67 (KLEPTOSE® CRYSMEB, ROQUETTE FRERES), ci-après désignée « MβCD-0,67 », une méthyle-β-cyclodextrine présentant un MS de 1,8 (RAMEB®, CYCLOLAB), ci-après désignée « MβCD-1,8 ».

### Incubation des cellules avec les cyclodextrines :

0.5 µCi/mL de H3-cholestérol a été incorporé dans du sérum (10% sérum de cheval/veau (HS/CS)) pendant 6 heures à 37 °C dans le but d'obtenir un milieu de culture enrichi en cholestérol radioactif. Après cette incorporation, le milieu de culture a été finalisé par incorporation de DMEM (Dulbecco's Modified Eagle's médium) supplémenté avec 10%HS, 10%CS, bFGF (basic fibroblast growth factor) 1ng/mL, glutamine 2mM et Gentamicine 50µg/mL.
Les cellules ont été ensuite cultivées dans ce milieu radioactif pendant 36 heures pour leur permettre d'incorporer le cholestérol radioactif. Le milieu a ensuite été éliminé et les cellules rincées deux fois avec du DMEM chaud contenant 0.1% de sérum albumine bovine (SAB).
Les cellules ont ensuite été cultivées en DMEM/bFGF 1ng/mL contenant 1 mM de chaque cyclodextrine pendant 24 heures (1,4 mL/puits).

### A. Mesure du cholestérol capté par les cyclodextrines

Dans cet exemple, la Demanderesse a étudié l'effet des cyclodextrines sur les efflux de cholestérol, en fonction de leur degré de méthylation.
Deux heures après le début de l'incubation en présence de chaque cyclodextrine, 500 µL de milieu ont été prélevés et le dosage de cholestérol radioactif capté par la cyclodextrine a été effectué au moyen d'un compteur à scintillation (Tri-carb 2100TR). La radioactivité a été mesurée en désintégration par minute (DPM). La capacité de chaque cyclodextrine à capter le cholestérol cellulaire a alors été estimée en réalisant le calcul suivant : ((1,4 mL/0,5 mL)* DPM mesurées).
Les valeurs ont ensuite été converties sous forme de pourcentages qui sont comparés à la condition incubée sans cyclodextrine (contrôle) représentant le 100 %.
Chaque expérience a été réalisée en triplicat et répétée deux fois.
Les résultats obtenus sont présentés à la Figure 2.

### Résultats :

Les MβCD-0,2 et MβCD-0,67 conformes à l'invention provoquent un efflux de cholestérol nettement moins important que les βCD et MβCD-1,8 non conformes à l'invention.
Les inventeurs ont donc démontré une plus faible affinité vis-à-vis du cholestérol pour les cyclodextrines méthylées selon l'invention illustrant un efflux de cholestérol sous la forme de cholestérol-HDL et non sous une forme de cholestérol complexée à des cyclodextrines, ou à des vésicules de phospholipides.

### B. Expression des transporteurs responsables de l'efflux du cholestérol intracellulaire : ABCA1 et ABCG1

Dans cet exemple, la Demanderesse a étudié l'expression de deux transporteurs responsables des efflux de cholestérol, le récepteur ABCA1 et le récepteur ABCG1, suite à un traitement par des cyclodextrines de différents degrés de méthylation.
Après 24 heures de traitement avec chaque cyclodextrine, les cellules ont été rincées deux fois en PBS froid (8 g/L NaCl, 0,2 g/L KCl, 0,2 g/L KH₂PO₄, 2,87 g/L NA₂HPO₄ (12H₂O), pH 7.4) et lysées grâce à 500 µL de tampon de lyse RLT (Biorad). Trois puits ont été utilisés et poolés pour chaque condition. La purification des ARN messagers (ARNm) a été effectuée en utilisant le kit RNeasy total RNA extraction kit (Qiagen) en suivant les recommandations du fabricant. A la fin de cette étape de purification, la concentration des ARNm a été estimée grâce aux valeurs d'absorbance obtenues à 260, 280 et 320 nm. La pureté des échantillons a été vérifiée en réalisant le rapport 260/280 et seuls les échantillons présentant une valeur supérieure à 2 ont été considérés pour nos études.
Pour chaque échantillon, les cDNA ont été synthétisés à partir de 250 ng d'ARNm en utilisant le kit iScript Reverse Transcription Supermix (Biorad) en suivant les instructions du fabricant. Les expériences de PCR en temps réel ont été effectuées en utilisant le kit Sso Fast EvaGreen Master Mix (BioRad) et les amorces dessinées et optimisées par la Demanderesse (Tableau 3).

**Tableau 3 :**

| ARNm | Espèce | Séquences | NO d'Accession |
|---|---|---|---|
| ABCA1 | Bos taurus | F 5'-gtgtctcgcctgttctcag-3' (SEQ ID No 1) | NM_001024693 |
| | | R 5'-gaaacatcacctcctgccg-3' (SEQ ID No 2) | |
| ABCG1 | Homo sapiens | F 5'-gaggaagaaaggatacaagacc-3' (SEQ ID No 3) | BC029158 |
| | | R 5'-gtcagtatctccttgaccattt-3' (SEQ ID No 4) | |

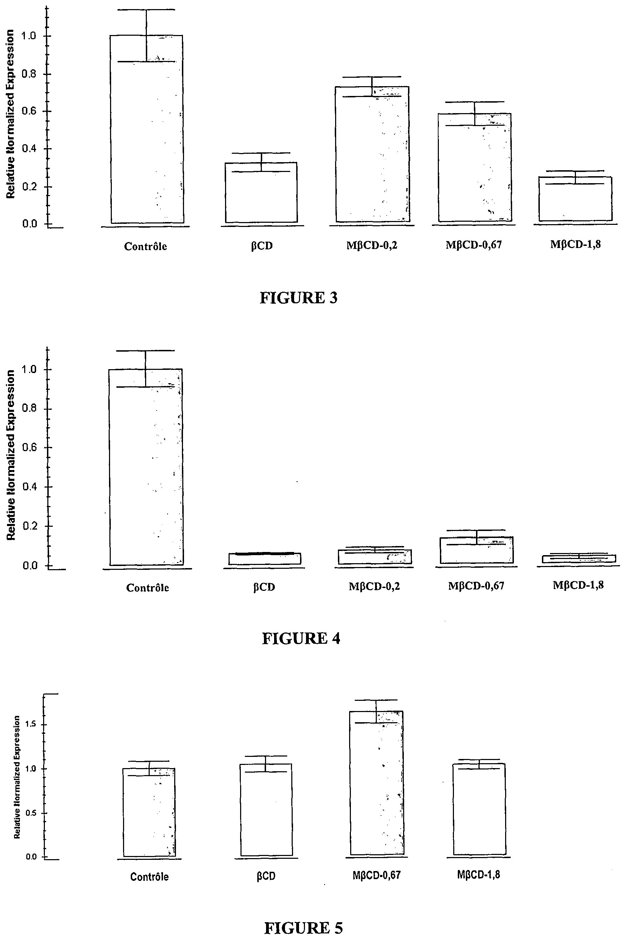

L'amplification a été réalisée sur 40 cycles avec une température d'hybridation de 60 °C en utilisant le thermocycler CFX96 de Biorad. Pour chaque couple d'amorces, la spécificité d'amplification a été vérifiée en effectuant des courbes de fusion et l'efficacité a été déterminée en réalisant des courbes de dilution. Les niveaux d'expression mesurés ont ensuite été normalisés sur les niveaux d'expression de la β-actine et la cyclophiline B en utilisant la méthode des ΔΔCt.
Chaque expérience a été réalisée en triplicat et répétée deux fois.
Les résultats obtenus sont présentés aux Figures 3 et 4.

### Résultats :

Les résultats montrent qu'en réponse à un traitement par une cyclodextrine, de manière générale, l'expression des récepteurs ABCA1 et ABCG1 par les cellules endothéliales diminue comparativement au contrôle.
En particulier, les MβCD-0,2 et MβCD-0,67 selon l'invention provoquent une diminution de l'expression des récepteurs ABCA1 et ABCG1 inférieure, comparativement aux βCD et MβCD-1,8.
En combinant ces résultats avec ceux obtenus ci-dessus (point A), les inventeurs ont montré que les βCD et MβCD-1,8 non conformes à l'invention induisaient un efflux rapide et important de cholestérol et une réaction plus vive de la cellule en diminuant fortement l'expression de récepteurs ABCA1 et ABCG1.
En revanche, les MβCD-0,2 et MβCD-0,67 conformes à l'invention, même si elles induisent des efflux inférieurs de cholestérol, diminuent bien moins nettement l'expression des récepteurs ABCA1 et ABCG1.
La cinétique d'efflux du cholestérol des cyclodextrines méthylées conformes à l'invention est ainsi totalement différente de celle des cyclodextrines non conformes à l'invention. Il est postulé par les inventeurs que cette cinétique particulière des cyclodextrines méthylées conformes à l'invention présente l'avantage de moins perturber l'homéostasie du cholestérol cellulaire.

### C. Etude de l'expression du récepteur SR-B1 (« Scavenger Receptor class B member 1 »), récepteur aux HDL, de cellules endothéliales traitées avec des cyclodextrines

Dans cet exemple, la Demanderesse a étudié l'effet des cyclodextrines sur l'expression d'un récepteur aux HDL, le récepteur SR-B1, en fonction de leur degré de méthylation. Un des rôles des récepteurs SR-B1 au niveau de l'endothélium, est de promouvoir l'efflux de cholestérol libre vers les HDL de la circulation.

L'expression du récepteur SR-B1 a été étudiée selon le protocole tel que décrit précédemment, en utilisant des amorces dessinées et optimisées par la Demanderesse (Tableau 4).

**Tableau 4 :**

| **ARNm** | **Espèce** | **Séquences** | **NO d'Accession** |
|---|---|---|---|
| SCARB1 | Bos taurus | F 5'-cccttaatccacctcatcaatc-3' (SEQ ID No 5) | NM_174597 |
| | | R 5'-gaagtttttgacccctgtgaac-3' (SEQ ID No 6) | |

Chaque expérience a été réalisée en triplicat et répétée deux fois.
Les résultats obtenus sont présentés Figure 5.

### Résultats :

La MβCD-0,67 selon l'invention provoque une forte augmentation de l'expression du récepteur SR-B1, d'environ 50% de l'expression basale.

En conclusion, les inventeurs ont montré que les méthyl-cyclodextrines selon la présente invention présentaient une faible affinité pour le cholestérol, limitaient la diminution des transporteurs responsables de l'efflux de cholestérol et augmentaient l'expression du récepteur SR-B1 aux HDL, ceci ayant pour effet de favoriser le transport du cholestérol cytoplasmique vers le sang et donc d'augmenter le taux de cholestérol plasmatique, en particulier le cholestérol-HDL.
Les inventeurs ont donc démontré que méthyl-cyclodextrines selon l'invention en dépit de leur faible affinité pour le cholestérol ne nécessitaient pas l'addition de vésicules de phospholipides pour exercer leurs effets illustrés par les résultats in vivo de l'exemple 1 (Tableaux 1 et 2).
Ces résultats renforcent l'intérêt des méthyl-cyclodextrines selon l'invention, pour une utilisation dans le traitement et/ou la prévention de maladies susceptibles d'être traitées et/ou prévenues par une augmentation du taux de cholestérol-HDL.

## Revendications

1. Composition pharmaceutique comprenant au moins une méthyl-cyclodextrine présentant un degré de substitution molaire compris entre 0,4 et 0,9, pour son utilisation dans le traitement et/ou la prévention de maladies susceptibles d'être traitées et/ou prévenues par une augmentation du taux de cholestérol-HDL.

2. Composition pharmaceutique comprenant au moins une méthyl-cyclodextrine présentant un degré de substitution molaire compris entre 0,4 et 0,9, pour son utilisation dans le traitement et/ou la prévention de l'athérosclérose ou des complications liées à un athérome.

3. Composition pharmaceutique pour son utilisation selon la revendication 1 ou 2, dans le traitement et/ou la prévention des complications liées à un athérome qui comprennent l'ischémie, de préférence l'ischémie du myocarde, les maladies coronariennes, l'angine de poitrine, le syndrome coronarien aigu, l'infarctus du myocarde, l'infarctus mésentérique, l'accident vasculaire-cérébral, l'anévrisme ou l'artériopathie des membres inférieurs et leurs conséquences.

4. Composition pharmaceutique pour son utilisation selon l'une quelconque des revendications 1 à 3, **caractérisée en ce que** la méthyl-cyclodextrine présente un degré de substitution molaire compris entre 0,6 et 0,8.

5. Composition pharmaceutique pour son utilisation selon l'une quelconque des revendications 1 à 4, **caractérisée en ce que** la méthyl-cyclodextrine est une méthyl-β-cyclodextrine.

6. Composition pharmaceutique pour son utilisation selon l'une quelconque des revendications 1 à 5, **caractérisée en ce que** la méthyl-cyclodextrine est substituée sur l'hydroxyle porté par le carbone C2 des unités glucopyranoses, ou par les carbones C3 et/ou C6 des unités glucopyranoses, ou par une combinaison des carbones C2, C3 et/ou C6, de préférence C2 et C6 des unités glucopyranoses.

7. Composition pharmaceutique pour son utilisation selon l'une quelconque des revendications 1 à 6, **caractérisée en ce que** la composition de méthyl-cyclodextrines comprend une ou plusieurs méthyl- β-cyclodextrines choisies parmi le groupe consistant en des méthyl-β-cyclodextrines substituées sur l'hydroxyle porté par le carbone C2 des unités glucopyranoses, des méthyl-β-cyclodextrines substituées sur l'hydroxyle porté par les carbones C3 et/ou C6 des unités glucopyranoses, des méthyl-β-cyclodextrines substituées sur l'hydroxyle porté par les carbones C2, C3 et/ou C6, de préférence C2 et C6 des unités glucopyranoses et lesdites méthyl- β-cyclodextrines présentant un degré de substitution molaire compris entre 0,6 et 0,8.

8. Composition pharmaceutique pour son utilisation selon l'une quelconque des revendications 1 à 7, **caractérisée en ce que** la composition de méthyl-cyclodextrines comprend au moins 50, 60, ou 75 % de méthyles substituées sur l'hydroxyle porté par le carbone C2 des unités glucopyranoses.

9. Composition pharmaceutique pour son utilisation selon l'une quelconque des revendications 1 à 8, **caractérisée en ce que** la composition comprenant au moins une méthyl-cyclodextrine comprend également une cyclodextrine, en particulier β - cyclodextrine, non substituée et/ou une cyclodextrine, en particulier β -cyclodextrine, substituée par des groupes sulfobutyléther (SBE-) ou hydroxypropyles (HP-), de préférence avec un degré de substitution molaire compris entre 0,05 et 1,5.

10. Composition pharmaceutique pour son utilisation selon l'une quelconque des revendications 1 à 9 comprenant en outre au moins un agent actif supplémentaire, de préférence, un agent actif utilisé dans le traitement de l'athérosclérose ou des complications liés à un athérome, préférentiellement,
- une statine,
- un agent antiagrégant plaquettaire ou anticoagulant, de préférence sélectionné parmi l'aspirine, le clopidogrel, les nouveaux anticoagulants oraux comme le dabigatran, l'apixaban, le rivaroxaban, et les anti-vitamines K,
- un agent antihypertenseur, de préférence sélectionné parmi les inhibiteurs de l'enzyme de conversion de l'angiotensine comme le perindopril, le captopril, l'enalapril, le lisinopril ou le ramipril, les antagonistes du récepteur à l'angiotensine II comme le losartan, le valsartan, ou le candesartan, et et/ou parmi les bêta-bloquants comme l'acébutolol, le labétalol, le nadolol, l'oxprénolol, le penbutolol, le pindolol, ou le propranolol,
- ou une combinaison de ceux-ci.

11. Composition pharmaceutique pour son utilisation selon la revendication 10, **caractérisée en ce que** l'agent actif supplémentaire est un antagoniste du récepteur à l'angiotensine II, comme le losartan, le valsartan, ou le candesartan.

12. Composition pharmaceutique pour son utilisation selon l'une quelconque des revendications 1 à 11, **caractérisée en ce que** la composition est susceptible d'être administrée par administration par voie orale, par voie parentérale, ou par voie cutanée ou mucosale.

13. Composition pour son utilisation selon l'une quelconque des revendications 1 à 12, **caractérisée en ce que** ladite composition est exempte de vésicules de phospholipides.

## Patentansprüche

1. Pharmazeutische Zusammensetzung, umfassend mindestens ein Methyl-Cyclodextrin, das einen molaren Substitutionsgrad zwischen 0,4 und 0,9 aufweist, zur Verwendung bei der Behandlung und/oder Vorbeugung von Krankheiten, die durch eine Erhöhung des HDL-Cholesterinspiegels behandelt und/oder denen dadurch vorgebeugt werden kann.

2. Pharmazeutische Zusammensetzung, umfassend mindestens ein Methyl-Cyclodextrin, das einen molaren Substitutionsgrad zwischen 0,4 und 0,9 aufweist, zur Verwendung bei der Behandlung und/oder Vorbeugung von Atherosklerose oder Komplikationen, die mit einem Atherom verbunden sind.

3. Pharmazeutische Zusammensetzung zur Verwendung nach Anspruch 1 oder 2, bei der Behandlung und/oder Vorbeugung von Komplikationen, die mit einem Atherom verbunden sind, umfassend Ischämie, vorzugsweise Myokardischämie, koronare Erkrankungen, Angina pectoris, akutes Koronarsyndrom, Myokardinfarkt, Magen-Darm-Infarkt, Schlaganfall, Aneurysma oder Arteriopathie der unteren Gliedmaßen und ihre Folgen.

4. Pharmazeutische Zusammensetzung zur Verwendung nach einem der Ansprüche 1 bis 3, **dadurch gekennzeichnet, dass** das Methyl-Cyclodextrin einen molaren Substitutionsgrad zwischen 0,6 und 0,8 aufweist.

5. Pharmazeutische Zusammensetzung zur Verwendung nach einem der Ansprüche 1 bis 4, **dadurch gekennzeichnet, dass** das Methyl-Cyclodextrin ein Methyl-β-Cyclodextrin ist.

6. Pharmazeutische Zusammensetzung zur Verwendung nach einem der Ansprüche 1 bis 5, **dadurch gekennzeichnet, dass** das Methyl-Cyclodextrin substituiert ist am Hydroxyl, das getragen wird vom Kohlenstoff C2, durch Glucopyranose-Einheiten, oder von den Kohlenstoffen C3 und/oder C6, durch Glucopyranose-Einheiten, oder von einer Kombination der Kohlenstoffe C2, C3 und/oder C6, vorzugsweise C2 und C6, durch Glucopyranose-Einheiten.

7. Pharmazeutische Zusammensetzung zur Verwendung nach einem der Ansprüche 1 bis 6, **dadurch gekennzeichnet, dass** die Zusammensetzung von Methyl-Cyclodextrinen ein oder mehrere Methyl-β-Cyclodextrine umfasst, die ausgewählt sind aus der Gruppe bestehend aus Methyl-β-Cyclodextrinen, substituiert am Hydroxyl, das getragen wird vom Kohlenstoff C2, durch Glucopyranose-Einheiten, Methyl-β-Cyclodextrinen, substituiert am Hydroxyl, das getragen wird von den Kohlenstoffen C3 und/oder C6, durch Glucopyranose-Einheiten, Methyl-β-Cyclodextrinen, substituiert am Hydroxyl, das getragen wird von den Kohlenstoffen C2, C3 und/oder C6, vorzugsweise C2 und C6, durch Glucopyranose-Einheiten, und wobei die genannten Methyl-β-Cyclodextrine einen molaren Substitutionsgrad zwischen 0,6 und 0,8 aufweisen.

8. Pharmazeutische Zusammensetzung zur Verwendung nach einem der Ansprüche 1 bis 7, **dadurch gekennzeichnet, dass** die Zusammensetzung von Methyl-Cyclodextrinen mindestens 50, 60 oder 75 % Methyle umfasst, die substituiert sind am Hydroxyl, das vom Kohlenstoff C2 getragen wird, durch Glucopyranose-Einheiten.

9. Pharmazeutische Zusammensetzung zur Verwendung nach einem der Ansprüche 1 bis 8, **dadurch gekennzeichnet, dass** die Zusammensetzung, die mindestens ein Methyl-Cyclodextrin umfasst, auch ein Cyclodextrin umfasst, insbesondere unsubstituiertes β-Cyclodextrin, und/oder ein Cyclodextrin, insbesondere β-Cyclodextrin, substituiert durch Sulfobutylether- (SBE-) oder Hydroxypropyl- (HP-) Gruppen, vorzugsweise mit einem molaren Substitutionsgrad zwischen 0,05 und 1,5.

10. Pharmazeutische Zusammensetzung zur Verwendung nach einem der Ansprüche 1 bis 9, außerdem umfassend mindestens einen ergänzenden Wirkstoff, vorzugsweise einen Wirkstoff, der vorzugsweise bei der Behandlung von Atherosklerose oder Komplikationen, die mit einem Atherom verbunden sind, verwendet wird,
- ein Statin,
- ein Mittel gegen die Plättchenaggregation oder ein Antikoagulans, vorzugsweise ausgewählt aus Aspirin, Clopidogrel, neuen oralen Antikoagulantien, wie Dabigatran, Apixaban, Rivaroxaban und Anti-Vitaminen K,
- ein Antihypertensivum, vorzugsweise ausgewählt aus Inhibitoren des Enzyms zur Umwandlung von Angiotensin, wie Perindopril, Captropril, Enalapril, Lisinopril oder Ramipril, Antagonisten des Angiotensin II-Rezeptors, wie Losartan, Valsartan oder Candesartan, und/oder aus Betablockern, wie Acebutolol, Labetalol, Nadolol, Oxprenolol, Prenbutolol, Pindolol oder Propanolol,
- oder eine Kombination derselben.

11. Pharmazeutische Zusammensetzung zur Verwendung nach Anspruch 10, **dadurch gekennzeichnet, dass** der ergänzende Wirkstoff ein Antagonist des Angiotensin II-Rezeptors ist, wie Losartan, Valsartan oder Candesartan.

12. Pharmazeutische Zusammensetzung zur Verwendung nach einem der Ansprüche 1 bis 11, **dadurch gekennzeichnet, dass** die Zusammensetzung durch Verabreichung auf oralem Weg, auf parenteralem Weg oder auf kutanem oder mukosalem Weg verabreicht werden kann.

13. Pharmazeutische Zusammensetzung zur Verwendung nach einem der Ansprüche 1 bis 12, **dadurch gekennzeichnet, dass** die genannte Zusammensetzung keine Phospholipidvesikel aufweist.

## Claims

1. A pharmaceutical composition comprising at least one methyl-cyclodextrin having a degree of molar substitution between 0.4 and 0.9, for use in the treatment and/or prevention of diseases suitable for being treated and/or prevented with an increase in the HDL cholesterol level.

2. The pharmaceutical composition comprising at least one methyl-cyclodextrin having a degree of molar substitution between 0.4 and 0.9, for use in the treatment and/or prevention of atherosclerosis or atheroma-related complications.

3. The pharmaceutical composition for use according to claim 1 or 2, in the treatment and/or prevention of atheroma-related complications which include ischaemia, preferably myocardial ischaemia, coronary diseases, angina pectoris, acute coronary syndrome, myocardial infarction, mesenteric infarction, stroke, aneurysm or arteriopathy of the lower limbs and the consequences thereof.

4. The pharmaceutical composition for use according to any one of claims 1 to 3, **characterized in that** the methyl-cyclodextrin has a degree of molar substitution between 0.6 and 0.8.

5. The pharmaceutical composition for use according to any one of claims 1 to 4, **characterized in that** the methyl-cyclodextrin is a methyl-β-cyclodextrin.

6. The pharmaceutical composition for use according to any one of claims 1 to 5, **characterized in that** the methyl-cyclodextrin is substituted by the hydroxyl carried by the C2 carbon of the glucopyranose units, or by the C3 and/or C6 carbons of the glucopyranose units, or by a combination of the C2, C3 and/or C6, preferably C2 and C6, carbons of the glucopyranose units.

7. The pharmaceutical composition for use according to any one of claims 1 to 6, **characterized in that** the methyl-cyclodextrin composition comprises one or more methyl-β-cyclodextrins chosen among the group consisting of methyl-β-cyclodextrins substituted on the hydroxyl carried by the C2 carbon of the glucopyranose units, methyl-β-cyclodextrins substituted on the hydroxyl carried by the C3 and/or C6 carbons of the glucopyranose units, methyl-β-cyclodextrins substituted on the hydroxyl carried by the C2, C3 and/or C6, preferably C2 and C6, carbons of the glucopyranose units and said methyl-β-cyclodextrins having a degree of molar substitution between 0.6 and 0.8.

8. The pharmaceutical composition for use according to any one of claims 1 to 7, **characterized in that** the methyl-cyclodextrin composition comprises at least 50, 60, or 75% of methyls substituted on the hydroxyl carried by the C2 carbon of the glucopyranose units.

9. The pharmaceutical composition for use according to any one of claims 1 to 8, **characterized in that** the composition comprising at least one methyl-cyclodextrin also comprises a non-substituted cyclodextrin, in particular β-cyclodextrin, and/or a cyclodextrin, in particular β-cyclodextrin, substituted by sulfobutylether (SBE-) or hydroxypropyl (HP-) groups, preferably with a degree of molar substitution between 0.05 and 1.5.

10. The pharmaceutical composition for use according to any one of claims 1 to 9 further comprising at least one additional active agent, preferably, an active agent used in the treatment of atherosclerosis or atheroma-related complications, preferentially,
- a statin,
- an anti-platelet aggregation or anticoagulant agent, preferably selected among aspirin, clopidogrel, new oral anticoagulants such as dabigatran, apixaban, rivaroxaban, and vitamin K antagonists,
- an anti-hypertensive agent, preferably selected among angiotensin-converting enzyme inhibitors such as perindopril, captopril, enalapril, lisinopril or ramipril, angiotensin II receptor antagonists such as losartan, valsartan, or candesartan, and and/or among beta-blockers such as acebutolol, labetalol, nadolol, oxprenolol, penbutolol, pindolol, or propranolol,
- or a combination thereof.

11. The pharmaceutical composition for use according to claim 10, **characterised in that** the additional active agent is an angiotensin II receptor antagonist, such as losartan, valsartan, or candesartan.

12. The pharmaceutical composition for use according to any one of claims 1 to 11, **characterised in that** the composition is suitable for being administered orally, parenterally, or by the cutaneous or mucosal route.

13. A composition for use according to any one of claims 1 to 12, **characterized in that** said composition is free from phospholipid vesicles.
